**Europäisches Patentamt**

**European Patent Office**

**Office européen des brevets**

(11) Veröffentlichungsnummer: **0 344 314**
**A1**

(12) # EUROPÄISCHE PATENTANMELDUNG
Veröffentlicht nach Art. 158 Abs. 3 EPÜ

(21) Anmeldenummer: 88902618.3

(22) Anmeldetag: 26.11.87

Daten der zugrundeliegenden internationalen Anmeldung:

(86) Internationale Anmeldenummer:
PCT/SU87/00138

(87) Internationale Veröffentlichungsnummer:
WO89/04864 (01.06.89 89/12)

(51) Int. Cl.³: **C 12 M 1/34**

(43) Veröffentlichungstag der Anmeldung:
**06.12.89 Patentblatt 89/49**

(84) Benannte Vertragsstaaten:
**AT CH DE FR GB LI SE**

(71) Anmelder: **KAUNASSKY POLITEKHNICHESKY INSTITUT IMENI ANTANASA SNECHKUSA**
**ul. Donelaichio, 73**
**Kaunas, 233006(SU)**

(72) Erfinder: **ZHALKAUSKAS, Vitautas Antanovich**
**ul. K.Marxa, 37-81**
**Vilnjus, 232000(SU)**

(72) Erfinder: **STANISHKIS, Jurgis-Kazimeras Jurgevich**
**ul. Pleno, 1-11**
**Kaunas, 233035(SU)**

(72) Erfinder: **BYARULIS, Donatas Eduardovich**
**ul. Aushros, 79-1**
**Kaunas, 233005(SU)**

(72) Erfinder: **SIMUTIS, Rimvidas Juozovich**
**ul. Kurshju, 18-60**
**Kaunas, 233031(SU)**

(72) Erfinder: **KONDRATAVICHJUS, Mindaugas Vatslovovich**
**ul. Partizanu, 186-26**
**Kaunas, 233030(SU)**

(74) Vertreter: **Nix, Frank Arnold, Dr.**
**Kröckelbergstrasse 15**
**D-6200 Wiesbaden(DE)**

(54) **VORRICHTUNG ZUR MESSUNG DER GESCHWINDIGKEIT DES SAUERSTOFFVERBRAUCHS VON MIKROORGANISMEN IN EINEM FLÜSSIGEN MEDIUM.**

(57) Die erfindungsgemässe Messanlage zur Messung der Schnelligkeit des Sauerstoffverbrauches von Mikroorganismen in flüssigen Medien enthält eine Durchflusskammer (1), electrochemische Geber des Sauerstoffpartialdrucks an dem Eingang (A) und an dem Ausgang (B) der Durchflusskammer (1) und eine Berechnungseinheit (20) zur Berechnung der Schnelligkeit der Sauerstoffverbrauches. Die elektrochemischen Geber sind in Form einer einheitlichen Baugruppe ausgeführt, die eine Kapsel (5) mit halbdurchlässigen Membranen (6, 7), die mit Elektrolyt ausgefüllt ist, zwei Katoden (11, 12) und eine Anode (13) innerhalb der Kapsel (5) einschliesst.

EP 0 344 314 A1

FIG. 1

# MESSANLAGE ZUR MESSUNG DER SCHNELLIGKEIT DES SAUERSTOFFVERBRAUCHES VON MIKROORGANISMEN IN FLÜSSIGEN MEDIEN

## Technisches Gebiet

Die vorliegende Erfindung bezieht sich auf das Gebiet Mikrobiologie, insbesondere auf Messanlage zur Messung der Schnelligkeit des Verbrauches von Sauerstoff durch Mikroorganismen in flüssigen Medien.

## Zugrundeliegender Stand der Technik

Bekannt ist eine Messanlage zur Messung der Schnelligkeit des Verbrauches an Sauerstoff von Mikroorganismen in flüssigen Medien, die Analysatoren der stationären Konzentrationen des Sauerstoffs als Bestandteil der in einen Fermenter ein- und aus diesem herausströmenden Gasmischung und eine Auswerteeinheit zur Ermittlung des Sauerstoffsverbrauchs durch die Mikroorganismen nach der Anzeigendifferenz dieser Analysatoren vorsieht ('Arch. Microbiol" 121, 3, 1979, Olijve, W., Kok J.J. "Analysis of growth of gluconobacter oxydans in glucose-containing media", S.S. 283 bis 290).

Diese bekannte Messanlage erfordert eine hochgenaue Messung der Zufuhrgeschwindigkeit der Gasmischung in den Fermenter, wozu kostspielige Geräte, Gasanalysatoren verwendet werden. Trotzdem sind die Empfindlichkeit und die Zuverlässigkeit der Ermittlung der Schnelligkeit des Verbrauches an Sauerstoff durch Mikroorganismen mit Hilfe dieser bekannten Messanlage niedrig, da die Anzeigendifferenz der Gasanalysatoren am Ein- bzw. Ausgang des Fermenters sehr gering ist.

Bekannt ist eine weitere Messanlage zur Messung der Schnelligkeit des Verbrauches an Sauerstoff durch Mikroorganismen in flüssigen Medien, die eine Durchflusskammer, ein Mittel zum Durchpumpen des flüssigen Kulturmediums durch die betreffende Kammer, das mit einem Sollwertgeber der Durchflussgeschwindigkeit ausgestattet ist, je einen am Ein- und Ausgang der Durchflusskammer angeordneten elektrochemischen Geber des Partialdrucks von Sauerstoff, eine Auswerteeinheit zur Ermittlung der Signaldifferenz an den Geberausgängen, die an diese Ausgänge der Geber angeschlossen ist, und eine Berechnungseinheit zur Schnelligkeitberechnung des Verbrauches an Sauerstoff durch Mikroorganismen einschliesst. Jeder elektrochemische Geber besteht aus einem Gehäuse, dessen eines Ende mit einer halbdurchlässigen Membrane geschlossen ist, und aus einer Katode mit einer Anode, die im Inneren des mit einem Elektrolyt gefüllten Gehäuse untergebracht sind. Das Kulturmedium mit den Mikroorganismen wird mit einer konstantbleibenden Strömungsgeschwindigkeit durch die Durchflusskammer hindurchgepumpt, während am Ein- und Ausgang der betreffenden Durchflusskammer vermittels der elektrochemischen Geber der Partialdruck des in diesem kulturellen Medium gelösten Sauerstoffs gemessen wird. Die Schnelligkeit des Sauerstoffverbrauches durch Mikroorganismen wird dabei als Differenz zwischen den Anzeigen von zwei, je einer am Ein- und Ausgang der Durchflusskammer angeordnet, elektrochemischen Gebern, geteilt durch die Durchflusszeit der kulturellen Flüssigkeit in der Durchflusskammer, ermittelt (US, A, 3 510 406).

Auch diese Messanlage wird durch einen hohen Gesamtfehler der Messung der Schnelligkeit des Sauerstoffverbrauches durch Mikroorganismen beeinträchtigt, der sich aus der Anwendung zweier einzelner elektrochemischer Geber des Partialdrucks des Sauerstoffs ergibt, deren Kennlinien keinenfalls identisch verlaufen können. Diese Kennlinien der elektrochemischen Geber hängen mit den quantitativen und qualitativen Kennwerten des Elektrolyten, der Anode sowie mit der Stärke der Elektrolytschicht zwischen der halbdurchlässigen Membrane und der Katode zusammen. Infolge der im Inneren des Gebers verlaufenden elektrochemischen Reaktionen aber treten quantitative und qualitative Veränderungen sowohl auf der Oberfläche der Anode als auch innerhalb des Elektrolyten auf. Diese Veränderungen wirken sich beim Geberbetrieb verschiedenartig aus. Darüber hinaus stellt die Herstellung von zwei einzelnen Gebern mit identischen Kennwerten der Elektrolytschicht zwischen der Katode und der halbdurchlässigen Membrane und Erzielung deren Stabilität in der Zeit eine komplizierte technische Aufgabe dar, wobei die Kennwerte dieser Elektrolytschicht sowohl die statischen als auch die dynamischen Kennlinien eines jeweiligen elektrochemischen Gebers bestimmen.

Offenbarung der Erfindung

Der vorliegenden Erfindung liegt die Aufgabe zugrunde, eine Messanlage zur Messung der Schnelligkeit des Sauerstoffverbrauches durch Mikroorganismen in flüssigen Medien zu entwickeln, in der die elektrochemischen Geber des Partialdrucks des Sauerstoffs am Ein- und Ausgang der Durchflusskammer derart ausgeführt sind, dass diese Geber identische Kennlinien aufweisen, welche von den quantitativen und qualitativen Kennwerten des Elektrolyten und der Anode unabhängig bleiben, was es ermöglicht, die Genauigkeit und Zuverlässigkeit der Messung der Schnelligkeit des Sauerstoffverbrauchs durch Mikroorganismen zu steigern.

Die gestellte Aufgabe wird dadurch gelöst, dass in der Messanlage zur Messung der Schnelligkeit des Sauerstoffverbrauchs durch Mikroorganismen in flüssigen Medien, die eine mit einem Sollwertsteller der Durchflussgeschwindigkeit ausgerüstete Durchpumpvorrichtung zum Durchpumpen des kulturellen Mediums durch die Durchflusskammer, die je einen am Ein- und Ausgang der Durchflusskammer angeordneten elektrochemischen Geber des Partialdrucks des Sauerstoffs, eine an den Ausgängen der elektrochemischen Geber angeschlossene Auswerteeinheit der Differenz der von den Gebern ausgehenden Signale und eine an den Ausgang der Auswerteeinheit der Signaldifferenz angeschlossene Berechnungseinheit zur Berechnung der Schnelligkeit des Sauerstoffverbrauches durch Mikroorganismen vorsieht, erfindungsgemäss die elektrochemischen Geber zur Ermittlung des Partialdrucks des Sauerstoffs in Form einer einzelnen Gesamteinheit ausgeführt sind, die eine Kapsel, in deren zwei gegenüberliegenden Wänden mit halbdurchlässigen Membranen verschlossene Öffnungen ausgespart sind, wobei die Kapsel mit Elektrolyt ausgefüllt ist, zwei Katoden, jede von denen innerhalb der Kapsel in der Nähe einer ihr entsprechenden Membrane untergebracht ist, eine ebenfalls im Inneren der Kapsel untergebrachte Anode, Stromableitungen der Katoden und der Anode, die hier Ausgänge der Geber darstellen, vorsieht, wobei der Innenraum der Kapsel über die halbdurchlässigen Membranen mit dem Innenraum der Durchflusskammer kommuniziert und die Kapsel selbst in bezug auf die Durchflusskammer derart angeordnet ist, dass eine Membrane in unmittelbarer Nähe des Einganges der Durchflusskammer und die andere Membrane in unmittelbarer Nähe deren Ausganges untergebracht sind.

Zweckmässigerweise ist ein Federungselement innerhalb der Kapsel zwischen den Katoden untergebracht, das sich in der Stellung der elastischen Verformung längs der zu den Ebenen der halbdurchlässigen Membranen senkrecht stehenden Achse befindet und mit den Katoden in mechanischer Verbindung steht sowie an diesen eine Kraftbelastung in Richtung des Herandrückens der Katoden zu den halbdurchlässigen Membranen ausübt.

Die Messanlage zur Messung der Schnelligkeit des Sauerstoffverbrauches durch Mikroorganismen in flüssigen Medien, die der vorliegenden Erfindung gemäss ausgeführt ist, zeichnet sich durch ihre hohe Messgenauigkeit aus.

Die Ausführung der elektrochemischen Geber in Form einer einzelnen Gesamteinheit, mit deren Hilfe die Differenz der Partialdrücke des in dem Kulturmedium aufgelösten Sauerstoffs durch die in einen gemeinsamen Elektrolyt eingetauchten und eine gemeinsame Anode besitzenden Elektrodenpaare gemessen wird, ermöglicht es, die Unterschiedlichkeit der qualitativen und quantitativen Kennwerte an der Oberfläche der Anode und des Elektrolyten zu vermeiden. Durch die Zusammenkoppelung der Katoden über die mechanische Verbindung vermittels des elastischen Bauelements wird die Bildung von dünnen Elektrolytschichten zwischen den Katoden und halbdurchlässigen Membranen mit identischen Kennwerten erzielt, die die Differenz zwischen den Kennlinien der Elektrodenpaare zu eliminieren ermöglichen, die sonst bei der Anwendung von zwei vereinzelten, elektrochemischen Gebern vorhanden sind.

Kurzbeschreibung der Zeichnungen

Nachstehend wird die Erfindung an Hand der Beschreibung konkreter Ausführungsvarianten und der angelegten Zeichnungen näher erläutert; in diesen zeigt:

Fig. 1 erfindungsgemässe Messanlage zur Messung der Schnelligkeit des Sauerstoffverbrauches von Mikroorganismen in flüssigen Medien in schematischer Darstellung mit teilweisem Längsschnitt;

Fig. 2 erfindungsgemässe Gesamteinheit der Geber des Partialdrucks des Sauerstoffs mit dem elastischen Federungselement im vergrösserten Maßstab, längsgeschnitten;

Fig. 3 Katoden der Geber des Sauerstoffpartialdrucks, die miteinander über ein Federungselement anderer Ausführung gekoppelt sind.

Bester Weg zur Ausführung der Erfindung

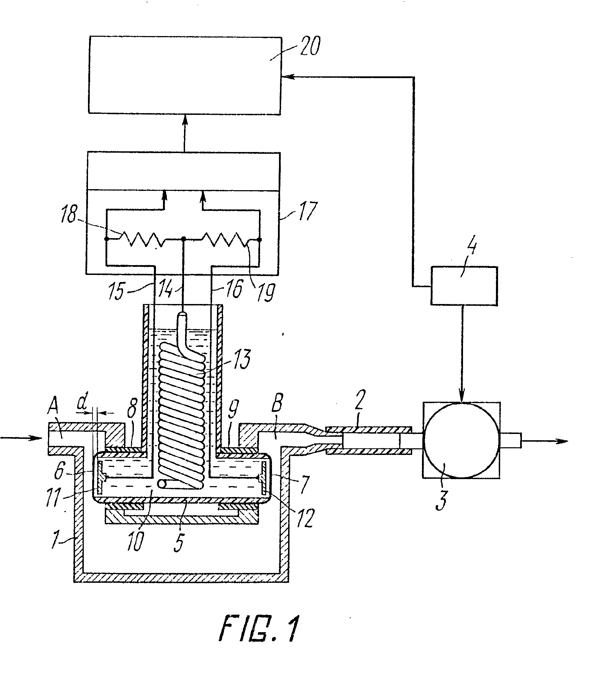

Die in der Fig. 1 dargestellte Messanlage zur Messung der Schnelligkeit des Sauerstoffverbrauches von Mikroorganismen in flüssigen Medien schliesst eine einen Eingang A und Ausgang B aufweisende Durchflusskammer 1 ein, die an ihrem Ausgang B vermittels Rohrstutzens 2 mit einer Durchpumpvorrichtung 3, beispielsweise einer peristaltischen Pumpe zum Durchpumpen des flüssigen Kulturmediums durch die Durchflusskammer 1, kommuniziert. Der Eingang A dient zum Anschliessen des aus der Zeichnung 1 nicht ersichtlichen Fermenters. Die Durchpumvorrichtung 3 ist mit einem Sollwertsteller 4 der Durchflussgeschwindigkeit bei Durchpumpen verbunden. Innerhalb der Durchflusskammer 1 ist eine beispielsweise aus Fluorkunststoff hergestellte Kapsel 5 untergebracht, die in zwei gegenüberliegenden Stirnwänden ausgesparte Öffnungen aufweist, welche durch den Innenraum der Kapsel 5 von dem Innenraum der Durchflusskammer 1 trennende , halbdurchlässige Membranen 6 und 7 geschlossen werden. Die halbdurchlässigen Membranen 6 und 7 können aus beispielsweise Teflon-, Polypropylen-, bzw. Polyäthylenfilm hergestellt werden. Die Kapsel 5 ist in der Durchflusskammer 1 vermittels der Abdichtungsringe 8 und 9 festgelegt, die zu gleicher Zeit auch als Befestigung für die halbdurchlässigen Membranen 6 und 7 dienen, wobei die Kapsel 5 derart angeordnet ist, dass sich die halbdurchlässige Membrane 6 in unmittelbarer Nähe an dem Eingang A in die Durchflusskammer 1 und die halbdurchlässige Membran 7 in unmittelbarer Nähe des Ausganges B der Durchflusskammer 1 befindet. Die Kapsel 5 ist mit einem Elektrolyt 10 ausgefüllt, während in der den halbdurchlässigen Membranen 6 und 7 nahen Gegend im Inneren der Kapsel 5 entsprechenderweise die Katoden 11 und 12 stehen, die Blättchen aus einem Edelmetall, beispielsweise aus Platin, Gold, Silber darstellen. Die betreffenden Katoden 11 und 12 sind derart angeordnet, dass zwischen jeder derselben und der entsprechenden halbdurchlässigen Membrane 6 und 7 eine dünne Schicht aus dem Elektrolyt gebildet wird. Darüber hinaus ist eine Anode 13 innerhalb der Kapsel 5 zwischen den erwähnten Katoden 11 und 12 symmetrisch eingesetzt, die beispielsweise aus einigen Windungen aus Blei- oder Kadmiumdraht bzw.-streifen gebildet ist. Die Anode 13 kann auch aus einigen Windungen eines mit einer AgCl-Schicht überzogenen Silberdrahts gebildet sein.

Als Geber des Sauerstoffpartialdruckes am Eingang A der Durchflusskammer 1 dient das Elektrodenpaar Katode 11 - Anode 13, während der Geber des Sauerstoffpartialdrucks am Ausgang B der Durchflusskammer 1 das Elektrodenpaar Katode 12 - Anode 13 ist. Die Anode 13 sowie die Katoden 11 und 12 sind mit Stromableitungen 14, 15 bzw. 16 ausgerüstet, die auch die Ausgänge der erwähnten elektrochemischen Geber darstellen, an welche die Auswerteeinheit 17 zur Ermittlung der Differenz der Gebersignale angeschlossen wird. Die

Auswerteeinheit 17 schliesst zwei in Differentialschaltung gekoppelte Widerstände ein und ist mit ihrem Ausgang an den Eingang der Berechnungseinheit 20 zur Berechnung der Schnelligkeit des Sauerstoffverbrauchs durch Mikroorganismen angeschlossen.

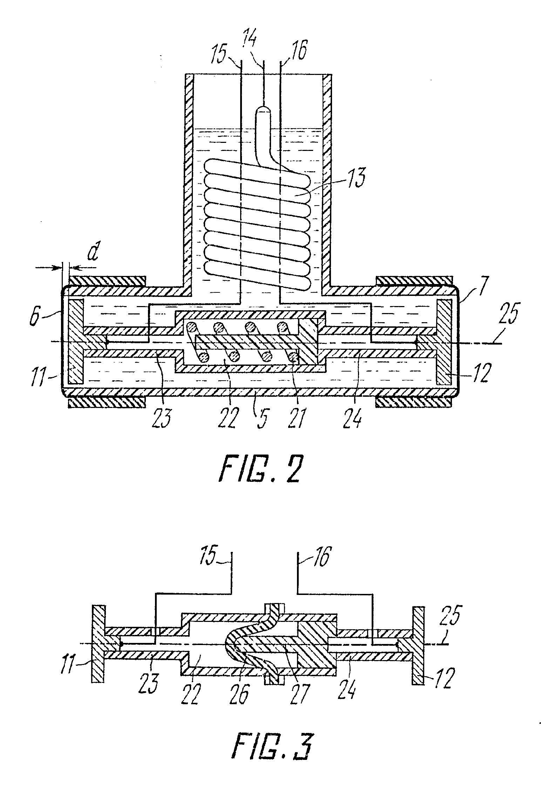

Die in Fig. 2 wiedergegebene Baugruppe der Geber des Sauerstoffpartialdrucks enthält im Unterschied von der entsprechenden in Fig. 1 dargestellten Baugruppe zusätzlich ein innerhalb der Kapsel 5 zwischen den Katoden 11 und 12 eingebautes Federungselement, vorliegend eine auf Zug arbeitende Zylinderfeder 21, die mit den Katoden 11 und 12 in mechanischer Verbindung steht und an diesen eine Kraftbelastung in Richtung des Herandrückens der Katoden 11, 12 an die halbdurchlässigen Membranen 6 bzw. 7 ausübt. Die Zylinderfeder 21 ist in der Höhle 22 eines an seinem Stirnende mit der Katode 11 starr verbundenen Stempels 23 untergebracht. Gleichzeitig steht ein anderer Stempel 24 an seinem einen Stirnende mit der Katode 12 in starrer Verbindung und wirkt mit seinem anderen Stirnende mit der Innenwandungsfläche der Höhle 22 des Stempels 23 gleitend zusammen und drückt gegen die Zylinderfeder 21, wodurch sich diese Zylinderfeder 21 im Zustand einer elastischen Verformung auf der zu den Ebenen der Membranen 6 und 7 senkrechten Achse 25 befindet.

In Fig. 3 ist eine weitere Variante der mechanischen Koppelung der Katoden 11, 12 vermittels der Stempel 23, 24 und eines Federungselementes wiedergegeben, das als eine Platte 26 aus einem elastischen Werkstoff, beispielsweise aus Gummi, ausgebildet ist, die in der Höhle 22 des Stempels 23 untergebracht und an deren Rändern in die Stempelwandung eingebaut ist. In diesem Falle trägt der Stempel 24 an seiner Stirnflanke einen mit der Platte 26 in Zusammenwirkung tretenden Vorsprung 27, durch welchen die Platte 26 längs der Achse 25 im Elastizitätsbereich verformt wird.

Die erfindungsgemässe Messanlage zur Messung der Schnelligkeit des Sauerstoffverbrauchs von Mikroorganismen in flüssigen Medien arbeitet wie folgt.

Ein Kulturmedium mit den Mikroorganismen wird mit der eingestellten Durchflussgeschwindigkeit Q aus dem Fermenter vermittels der Durchpumpvorrichtung 3 durch die Durchflusskammer 1 durchgepumpt. Die konstantbleibende Sollgeschwindigkeit des Durchpumpens wird an dem Sollwertsteller 4 der Durchpumpgeschwindigkeit eingestellt. Bei dem Durchfluss des Kulturmediums durch die Durchflusskammer 1 erfolgt die Absorption des in dem Kulturmedium gelösten Sauerstoffs durch die $V$ Mikroorganismen und dadurch auch die Verminderung des Partialdrucks des Sauerstoffs in diesem Medium. Dadurch bildet sich eine Differenz der Partialdruckgrössen des in dem Kulturmedium gelösten Sauerstoffs am Eingang A und dem Ausgang B der Durchflusskammer 1. Die Grösse dieser Druckdifferenz der Sauerstoffpartialdrücke ist bei einer konstant bleibenden Sollgeschwindigkeit Q des Durchpumpens der Schnelligkeit des Sauerstoffverbrauchs der Mikroorganismen proportional .

Der partielle Sauerstoffdruck am Eingang A der Durchflusskammer 1 wird mit Hilfe des aus der Katode 11 und der Anode 13 bestehenden Elektrodenpaars gemessen. Der in dem Kulturmedium aufgelöste Sauerstoff diffundiert durch die halbdurchlässige Membran 6, erreicht die dünne Elektrolytschicht d zwischen der Katode 11 und der Membran 6, wodurch an der Oberfläche der Katode 11, an der das negative Potential herrscht, der Prozess einer vollkommenen Reduktion des Sauerstoffs auf elektrischem Wege verläuft. Dieses negative Potential wird auf die Katode 11 durch die Verschliessung der Katode 11 mit der Anode 13 mit Hilfe der Stromableitungen 14 und 15 über den Widerstand 18 übermittelt. Dabei gleicht sich das Potential der Katode 11 mit dem Potential der Anode 13 aus. Der Werkstoff der Anode 13 wird derart gewählt, dass dadurch an der Anode 13 und der Katode 11 sich eine mit dem Potential der Reduktion des Sauerstoffs übereinstimmende Spannung entwickelt. Zu diesem Zweck kann beispielsweise Blei bzw. Kadmium ausgenutzt werden, d.h. Metalle mit einem verhältnismässig höheren negativen Potential. Falls als Anode 13 beispielsweise ein mit einer AgCl-Schicht überzogener Silberdraht ausgenutzt wird, wird dann das negative Potential an der Katode 11 vermittels einer Aussenspannungsquelle entwickelt, die zwischen der Katode 11 und der Anode 13 (aus der Zeichnung nicht ersichtlich) geschaltet wird.

Somit entwickelt sich infolge der elektrochemischen Reaktionen in der aus der Katode 11, der Anode 13 und dem Elektrolyt bestehenden Elektrodenzelle ein mit der Diffusionsgeschwindigkeit des Sauerstoffs durch die halbdurchlässige Membran 6 und mit dem Partialdruck des in dem Kulturmedium an dem Eingang A der Durchflusskammer 1 gelösten Sauerstoffs in direkter Proportionalitätsbeziehung zusammenhängender Diffusionssättigungsstrom. Bei dem Durchgang des Diffusionsstroms durch den Widerstand 18 tritt ein Spannungsabfall an diesem auf. Die Grösse dieses Spannungsabfalls steht ebenfalls mit dem Partialdruck des Sauerstoffs an dem Eingang A in die Durchflusskammer 1 in direkter Proportionalitätsbeziehung.

Ähnlicherweise wird der Partialdruck des Sauerstoffs auch an dem Ausgang B der Durchflusskammer 1 vermittels des aus der Katode 12 und der Anode 13 bestehenden Elektrodenpaars gemessen, wobei der Spannungsabfall an dem Widerstand 19 dem Partialdruck des Sauerstoffs an dem Ausgang B der Durchflusskammer 1 direkt proportional ist. Dadurch hängt die Differenz der Spannungsabfälle an den Widerständen 18 und 19 mit der Differenz der Partialdruckwerte des in dem Kulturmedium gelösten Sauerstoffes an dem Eingang A und an dem Ausgang B der Durchflusskammer 1 in direkter Proportionalitätsbeziehtung zusammen.

Diese Differenz ermittelt die Auswerteeinheit 17, von deren Ausgang ein Signal in die Berechnungseinheit 20 gelangt, in welcher die Berechnung der Schnelligkeit des Sauerstoffverbrauches der Mikroorganismen V nach folgender Beziehung durchgeführt wird:

$$r_{O_2} = K.Q.\Delta U/V,$$

in der $r_{O_2}$ die Schnelligkeit des Sauerstoffverbrauches der Mikroorganismen,

$\Delta U$ die Differenz der Signale der .Geber ,

Q die Durchflussgeschwindigkeit des Kulturmediums durch die Durchflusskammer 1,

V Rauminhalt der Durchflusskammer 1 und

K Proportionalitätsbeiwert bedeuten.

Die Ausführung der elektrochemischen Geber in Form einer einzelnen Gesamteinheit, in der die Elektroden in einen gemeinsamen Elektrolyten getaucht sind, die Anwendung einer gemeinsamen Anode für zwei Katoden ermöglicht es, den Unterschied zwischen den Kennlinien der Elektrodenpaare zu eliminieren, welcher durch die Veränderung der qualitativen und quantitativen Kennwerte des Elektrolyten und der Anodenoberfläche bei der Benutzung von zwei einzelnen elektrochemischen Gebern auftritt.

Die Veränderung der erwähnten Kennwerte tritt auch als Folge der innerhalb der Geber verlaufenden elektrochemischen Reaktionen sowie im Laufe der Sterilisierung der Geber bei hohen Temperaturwerten und auch infolge der jeweiligen Diffusion einiger Komponenten des Elektrolyten durch die halbdurchlässige Membran in das analysierbare Medium sowie durch die Diffusion einiger Stoffe aus dem analysierbaren Medium in den Elektrolyt auf. Bei

0344314

der Benutzung von zwei getrennten Gebern verändern die obenerwähnten Einflussfaktoren die Kennlinien, insbesondere die statischen Kennlinien der Geber in der Zeit unterschiedlich stark, was wesentliche Messfehler bei der Messung der Differenz der Partialdrücke des Sauerstoffs an dem Eingang A und an dem Ausgang B der Durchflusskammer 1 mit sich bringt.

Bei der Benutzung des erfindungsgemässen elektrochemischen Gebers beeinflussen diese Faktoren im Laufe der ganzen Betriebszeit die Kennwerte der Elektrodenpaare gleichmässig stark, wodurch die Möglichkeit besteht, den Messfehler bei der Messung der Differenz der Parteialdrücke des Sauerstoffs an dem Eingang A und an dem Ausgang B der Durchflusskammer 1 auszuschliessen und demzufolge die Genauigkeit der Messung der Schnelligkeit des Sauerstoffverbrauches durch die Mikroorganismen zu steigern.

Die Messanlage zur Messung der Schnelligkeit des Sauerstoffverbrauchs der Mikroorganismen mit der Baugruppe der Geber des Sauerstoffpartialdrucks in der Ausführungsvariante gemäss Fig. 2 arbeitet analog der in Fig. 1 wiedergegebenen Messanlage. Ein Unterschied besteht nur darin, daß in dieser Ausführungsvariante der Messanlage die Katoden 11 und 12 miteinander durch die mechanische Federungsverbindung mit Hilfe der Stempel 23, 24 und der Zylinderfeder 21 gekoppelt sind, die in elastischem Verformungszustand verbleibt, und ein gemeinsames bewegliches System bilden. Die in dem Elastizitätsverformungszustand befindliche Zylinderfeder 21 übt mit Hilfe der Stempel 23, 24 eine Kraftbelastung an den Katoden 11 und 12 aus, indem sie diese an der Achse 25 entlang in Richtung der halbdurchlässigen Membranen 6 bzw. 7 auseinanderstösst und die Katoden 11 und 12 an die Membranen 6 bzw. 7 andrückt Da die Katoden 11, 12, die Stempel 23, 24 und die Zylinderfeder 21 ein gemeinsames, bewegliches System bilden, werden die Katoden 11, 12 an die Membranen 6, 7 mit gleicher Kraft angedrückt, wodurch eine gleiche

0344314

Stärke der Elektrolytschichten d zwischen den Katoden 11 11 und 12 und den Membranen 6 bzw. 7 gesichert wird.

Die Ausführungsvariante der Messanlage, in der als Federungselement die Platte 26 aus einem elastischen Werkstoff benutzt wird, arbeitet ähnlicherweise mit dem Unterschied aber, dass gegebenenfalls das Federungselement auf Druck arbeitet.

Gewerbliche Verwendbarkeit

Die erfindungsgemässe Messanlage zur Messung der Schnelligkeit des Sauerstoffverbrauches von Mikroorganismen in flüssigen Medien kann in der medizinischen Industrie bei der Herstellung von Antibiotika und Vakzinen, in der Labensmittelindustrie bei der Fermentation der Lebensmittel, bei der biologischen Reinigung der Abwässer, in der Medizin bei den Diagnosestellungen von Erkrankungen, in der forschungswissenschaftlichen Praxis der biotechnologischen Vorgänge ihre Anwendung finden.

PATENTANSPRÜCHE

1. Messanlage zur Messung der Schnelligkeit des Sauerstoffverbrauches von Mikroorganismen in flüssigen Medien, die eine Durchflusskammer (1), eine mit einem Sollwertsteller (4) der Durchflussgeschwindigkeit versehene Durchpumpvorrichtung (3) zum Durchpumpen des flüssigen Kulturmediums durch die Durchflusskammer (1), elektrochemische, je einen an dem Eingang (A) und an dem Ausgang (B) der Durchflusskammer (1) angeordnete Geber des Sauerstoffpartialdrucks, eine Auswerteeinheit (17) zur Ermittlung der Differenz der Signale an den Ausgängen der Geber, die an diese Geberausgänge angeschlossen ist, und eine an den Ausgang der Auswerteeinheit (17) angeschlossene Berechnungseinheit (20) zur Berechnung der Schnelligkeit des Sauerstoffverbrauches der Mikroorganismen vorsieht, dadurch g e k e n n z e i c h n e t, dass die elektrochemischen Geber zur Ermittlung des Sauerstoffpartialdrucks in Form einer einheitlichen Baueinheit ausgeführt sind, die eine Kapsel (5), welche in zwei gegenüberliegenden Wänden ausgesparte, mit halbdurchlässigen Membranen (6, 7) überdeckte Öffnungen aufweist und mit Elektrolyt ausgefüllt ist, zwei innerhalb der Kapsel (5) jeweils in der Nähe einer zugehörigen Membrane (6, 7) untergebrachte Katoden (11, 12), eine ebenfalls innerhalb der Kapsel (5) untergebrachte Anode (13), Stromableitungen (15, 16, 14) der Katoden (11, 12) und der Anode (13) einschliesst, die die Ausgänge der Geber darstellen, wobei der Innenraum der Kapsel (5) über die halbdurchlässigen Membranen (6, 7) mit dem Innenraum der Durchflusskammer (1) kommuniziert und die Kapsel (5) selbst in bezug auf die Durchflusskammer (1) derart angeordnet ist, dass eine Membran (6) in unmittelbarer Nähe zu dem Eingang (A) der Durchflusskammer (1) und die andere Membran (7) in unmittelbarer Nähe zu deren Ausgang (B) angeordnet ist.

2. Messanlage nach Anspruch 1, dadurch g e k e n n-

z e i c h n e t, daß innerhalb der Kapsel (5) zwischen den Katoden (11, 12) ein Federungselement eingesetzt ist, das längs einer Achse (25) elastisch verformt ist, welche zu den Ebenen der halbdurchlässigen Membranen (6, 7) senkrecht verläuft, und das mit den Katoden (11, 12) in mechanischer Verbindung steht und an diesen eine Kraftbelastung in Richtung des Herandrückens der Katoden (11,12) an die halbdurchlässigen Membranen (6, 7) ausübt.

FIG.1

FIG. 2

FIG. 3

OO-20

0344314

## I. CLASSIFICATION OF SUBJECT MATTER (if several classification symbols apply, indicate all) [6]

According to International Patent Classification (IPC) or to both National Classification and IPC

IPC[4]    C 12 M 1/34

## II. FIELDS SEARCHED

### Minimum Documentation Searched [7]

| Classification System | Classification Symbols |
|---|---|
| IPC[4] | C 12 M 1/34, c 12 N 1/00, C 12 Q 1/00, G 01 N 27/40 |

### Documentation Searched other than Minimum Documentation
### to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | SU, A1, 487540 (Institut biokhimii i fiziologii mikroorganizmov AN SSSR et al.) 7 January 1987 (07.01.87), see column 3, lines 10-38 | 1,2 |
| A | SU, A1, 958495 (Moskovsky ordena Lenina, Ordina Oktyabrskoi Revoljutsii i ordena Trudovogo Krasnogo Znameni gosudarstvenny universitet im . M.V. Lomonosova) 15 September 1982 (15.09.82), see column 5, lines 5-56 | 1,2 |
| A | GB, A, 1359975, (BERGWERKSVERBAND GmbH), 17 July 1974 (17.07.74), see page 2, lines 80-130, page 3 lines 1-26 | 1,2 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 26 May 1988 (26.05.88) | 8 August 1988 (08.08.88) |

| International Searching Authority | Signature of Authorized Officer |
|---|---|
| ISA/SU | |